# EUROPEAN PATENT SPECIFICATION

(11) **EP 4 637 931 B1**
(45) Date of publication and mention of the grant of the patent: **09.12.2026**
(21) Application number: 23836466.5
(22) Date of filing: 20.12.2023
(51) Int. Cl.: A61Q 11/00, A61K 9/00, A61K 8/73, A61K 8/58, A61K 8/34, A61K 8/60, A61K 8/365, A61K 8/92, A61K 8/02, A61K 8/04, A61K 47/10, A61K 47/12, A61K 47/24, A61K 47/26, A61K 47/38, A61K 47/44, A61P 1/02

(54) **ORAL CARE COMPOSITION CONTAINING CELLULOSIC POLYMERS**
MUNDPFLEGEZUSAMMENSETZUNG ENTHALTEND CELLULOSEPOLYMEREN
COMPOSITION DE SOIN BUCCAL CONTENANT DES POLYMÈRES CELLULOSIQUES

(30) Priority: 21.12.2022 EP 22383254
(43) Date of publication of application: 29.10.2025
(73) Proprietor: Lacer, S.A., 08290 Cerdanyola del Vallès Barcelona (ES)
(72) Inventor: DEL CASTILLO NIETO, Juan Carlos, 08290 Cerdanyola del Vallès, Barcelona (ES); FERRÀ DOMINGO, Lourdes, 08290 Cerdanyola del Vallès, Barcelona (ES); FERRER SISO, Alicia, 08290 Cerdanyola del Vallès, Barcelona (ES); MARTÍN HERRERO, Cristina, 08290 Cerdanyola del Vallès, Barcelona (ES); MATA MOLINER, Montserrat, 08290 Cerdanyola del Vallès, Barcelona (ES); MIRA OTAL, Francisco Javier, 08290 Cerdanyola del Vallès, Barcelona (ES); RODRÍGUEZ AMIGO, Beatriz, 08290 Cerdanyola del Vallès, Barcelona (ES)
(74) Representative: ABG Intellectual Property Law, S.L.
(86) International application number: PCT/EP2023/086846
(87) International publication number: WO 2024/133393

(56) References cited:
- JP-A- 2000 319 153
- JP-A- H0 748 237
- JP-A- S59 101 417
- JP-B2- H0 772 125

## Description

### FIELD OF THE INVENTION

The invention relates to the field of oral care compositions, in particular to mouthwashes, tooth gels and toothpastes comprising cellulosic polymeric binders including hydroxypropyl methyl cellulose and antimicrobial agents including chlorhexidine and *o*-cymen-5-ol.

### BACKGROUND OF THE INVENTION

Oral hygiene is the practice of keeping the mouth clean, and is considered to be the best means of prevention of cavities (dental caries), gingivitis, periodontitis, and other dental and oral disorders such as bad breath (halitosis). Oral hygiene is necessary for all persons to maintain the health of their teeth and mouth. There are three key steps to regular dental health and maintenance: brushing, flossing and rinsing with a mouthwash. Teeth brushing involves the use of a toothpaste in conjunction with a toothbrush to help remove food debris and dental plaque. Mouthwashes are used for cleaning and freshening the mouth and for oral hygiene. Antiseptic and anti-plaque compounds are present in mouthwashes and toothpaste to kill germs that cause plaque, gingivitis, and bad breath.

Sensory-related aspects are an important contribution to consumers' acceptance and preference of oral care products. Examples of relevant sensory-related aspects for oral care products are appearance, sensory smoothness and granularity, and taste.

Mucoadhesivity of oral care compositions is an interesting property of oral care compositions which may enhance the effectivity of said compositions by increasing the time they remains in the area to be treated thereby prolonging the contact of the active components in the area upon use (see for example MaškováE. et al, Journal of Controlled Release, V. 324, 10 August 2020, pp 695-727). Cellulosic polymers have been used in the past to enhance bioadhesivity of compositions to mucosae (see WO 2005/014047 A1).

Antimicrobial agents are widely used in oral healthcare products, such as toothpastes and mouthwashes, to confer antimicrobial effect against different bacteria, which helps control the development of dental plaque, calculus formation, bleeding gums, and malodour. They may also help reduce the breath concentrations of volatile sulphur compounds (VSC), which are associated with halitosis, as well as having direct and indirect inhibitory effects on anaerobe mediated-VSC production. Therefore, the inclusion of antimicrobial compounds in oral care products is highly desirable.

One type of antibacterial compounds which have been proposed for use in oral care products are isopropylmethylphenols. These compounds penetrate deep-inside plaque and show high bactericidal effect [Park, Y.-D. et al., Int. J. Clin. Prev. Dent., 2010, 6(2), 55-61]. In particular o-cymen-5-ol has been proposed as an antibacterial to be used oral care products [Pizzey, R. L., Int. Dent. J., 2011, 61 (Suppl. 3), 33-40].

Chlorhexidine (CHX) and its salts have also found widespread use as a gold standard antimicrobial agent for the reduction of both plaque and gingivitis (Najafi MH et al., Dent Res J 2012;9:305-8).

Compositions comprising o-cymenol and a chlorhexidine salt are known in the art. For instance, JPH0748237A discloses a composition comprising o-cymenol and 0.01 wt % of chlorhexidine hydrochloride. This composition does not comprise a cellulosic polymer. The authors are further silent about the mucoadhesivity of the disclosed composition.

On the other hand, compositions comprising less than 60 wt% of water, a cellulosic polymer, chlorhexidine or salts thereof and o-cymenol have been disclosed in the art. For instance, JP2000319153 discloses a toothpaste composition with antibacterial effect which comprises, carboxymethylcellulose, o-cymenol and chlorhexidine hydrochloride. In addition, JPH0772125B2 discloses a composition with antibacterial effect which comprises, hydroxyethlethylcellulose, biosol and chlorhexidine hydrochloride. Also, JP S59101417A discloses a toothpaste composition comprising hydroxyethlethylcellulose, biosol and chlorhexidine hydrochloride. Each of these disclosed compositions comprise water in an amount of less than 60 wt%. The authors are silent about the mucoadhesivity of the disclosed compositions.

### SUMMARY OF THE INVENTION

The present inventors have found that the addition of chlorhexidine to oral compositions comprising cellulosic polymers tends to decrease the mucoadhesivity of said oral compositions but that further addition of o-cymen-5-ol to said compositions, which comprise both chlorhexidine or salts thereof and cellulosic polymers, mitigates the negative impact of chlorhexidine (or its salts) or even increases the mucoadhesivity of the compositions in comparison with those comprising cellulosic polymers but neither chlorhexidine (or its salts) nor *o*-cymen-5-ol.

The present inventors have also found that the addition of chlorhexidine to oral compositions comprising o-cymen-5-ol tends to decrease the mucoadhesivity of said oral compositions but that further addition of a cellulosic polymer to said compositions mitigates the negative impact of chlorhexidine (or its salts) or even increases the mucoadhesivity of the compositions in comparison with those comprising o-cymen-5-ol but neither chlorhexidine (or its salts) nor a cellulosic polymer.

Thus, in a first aspect, the present invention relates to an oral care composition comprising:
- a cellulosic polymer that is hydroxypropyl methylcellulose,
- o-cymen-5-ol,
   - from 0.05 wt% to 0.5 wt% of chlorhexidine or salts thereof expressed as chlorhexidine free base, and
   - water in an amount of at least 60 wt%.

In a second aspect, the present invention relates to an oral care composition as defined in the first aspect for use in the treatment and/or prevention of dental plaque formation, caries formation, calculus formation, halitosis, gingivitis, periodontitis, peri-implant mucositis and/or periimplantitis.

Herein is also disclosed the use of o-cymen-5-ol to increase the mucoadhesivity of compositions comprising a cellulosic polymer, in particular of compositions comprising both a cellulosic polymer and chlorhexidine or salts thereof.

### DETAILED DESCRIPTION OF THE INVENTION

In a first aspect, the present invention relates to an oral care composition comprising:
- a cellulosic polymer that is hydroxypropyl methylcellulose,
- o-cymen-5-ol,
- from 0.05 wt% to 0.5 wt% of chlorhexidine or salts thereof expressed as chlorhexidine free base, and
- water in an amount of at least 60 wt%.

The compositions of the invention are "oral care" compositions, which means that they are intended for topical use in the mouth, so any component present in the composition needs to be orally acceptable, that is, safe for topical use in the mouth in the amounts and concentrations provided.

The term "*o*-cymen-5-ol" refers to 4-isopropyl-3-methylphenol of formula (I).

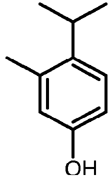

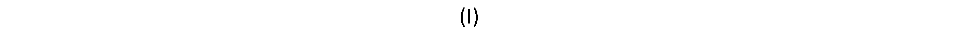

In a particular embodiment of the present invention o-cymen-5-ol is present at a concentration of from 0.005 to 1 wt%, preferably from 0.01 to 0.5 wt%, more preferably from 0.02 to 0.2 wt%, most preferably from 0.05 to 0.1 wt% with respect to the total weight of the composition.

The term "cellulosic polymer" is used in the present invention to designate any one of the following: hydroxyethylcellulose, carboxymethylcellulose (carmellose), hydroxypropyl methyl cellulose, hydroxypropylcellulose, ethyl cellulose, methylcellulose and salts thereof (e.g., carmellose sodium).

According to invention, the cellulosic polymer is hydroxypropyl methylcellulose.

In a particular embodiment of the present invention the cellulosic polymer is present at a concentration of from 0.05 to 4 wt% with respect to the total weight of the composition. The term "chlorhexidine" is used in the present invention to designate the compound of formula:

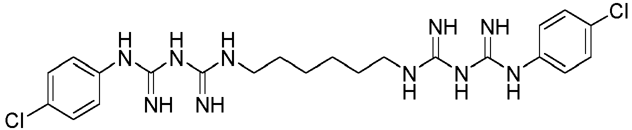

When the present invention refers to salts of chlorhexidine it is to be understood that any physiologically acceptable salt of the compound is included. These include, inter alia, the following: chlorhexidine gluconate, chlorhexidine digluconate, chlorhexidine acetate, chlorhexidine diacetate, chlorhexidine dihydrochloride, chlorhexidine phosphanilate (wherein phosphanilic acid is the compound shown below:

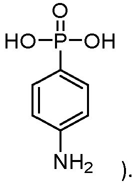

In a preferred embodiment chlorhexidine is used in the form of its salts, in particular chlorhexidine gluconate, chlorhexidine digluconate, chlorhexidine acetate, chlorhexidine diacetate, chlorhexidine dihydrochloride, more preferably chlorhexidine digluconate.

In a particular embodiment of the present invention the oral care compositions do not comprise any zinc salt having a solubility in water higher than 3.0·10⁴ mg/L at 25 °C. Examples of zinc salts having a solubility in water higher than 3.0·10⁴ mg/L at 25 °C are those belonging to the group consisting of zinc chloride, zinc sulphate such as the heptahydrate, zinc gluconate, zinc citrate, zinc acetate and mixtures thereof; preferably zinc chloride, zinc sulphate and zinc gluconate and mixtures thereof; more preferably zinc chloride, zinc gluconate and zinc acetate and mixtures thereof; more preferably zinc chloride, zinc gluconate and mixtures thereof; even more preferably zinc chloride.

In a particular embodiment of the present invention the oral care compositions do not comprise any glycyrrhizin derivative.

Examples of glycyrrhizin derivatives are those belonging to the groups consisting of glycyrrhizinic acid, glycyrrhetinic acid, salts thereof, and mixtures thereof.

"Glycyrrhizinic acid" or "glycyrrhizic acid" has the chemical structure depicted below.

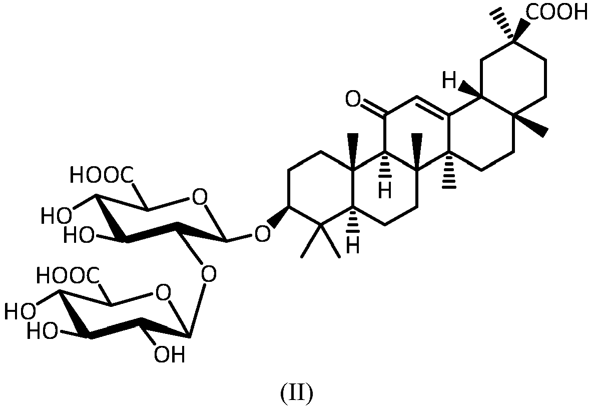

Glycyrrhizinic acid has three acid groups which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhizinic acid are potassium, sodium and ammonium. Examples of glycyrrhizinic acid salts are dipotassium glycyrrhizinate, diammonium glycyrrhizinate, disodium glycyrrhizinate, monoammonium glycyrrhizinate, monosodium glycyrrhizinate, and monopotassium glycyrrhizinate, preferably dipotassium glycyrrhizinate.

"Glycyrrhetinic acid" or "enoxolone" has the chemical structure depicted below.

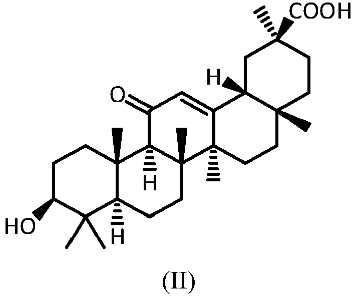

Glycyrrhetinic acid has one acid group which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhetinic acid are potassium, sodium and ammonium. Examples of glycyrrhetinic acid salts are potassium glycyrrhetinate, ammonium glycyrrhetinate, and sodium glycyrrhetinate.

In a particular embodiment of the present invention the oral care compositions do not comprise hydroxyacetophenone.

In a particular embodiment of the present invention the oral care compositions comprise water in an amount of at least 70 wt%, at least 75 wt%, or at least 80 wt%, wherein the wt% is expressed with respect to the total weight of the composition.

In a particular embodiment of the present invention the oral care compositions further comprise one or more ingredients selected from the group consisting of fluoride sources, surfactants, co-solvents, humectants, sweeteners, binders, mouth-feel agents, abrasives, desensitizing agents, other oral care active ingredients, pH modifying agents, other antimicrobial agents, chelating agents, flavoring agents, and colorants.

In a particular embodiment of the present invention the oral care compositions are in the form of a mouthwash, a tooth gel or a toothpaste.

In a particular embodiment of the present invention the oral care compositions are in the form of a mouthwash which comprises:
- 0.005 to 1 wt% of o-cymen-5-ol,
- 0.05 to 4 wt% of hydroxypropyl methylcellulose,
- 0.05 to 0.5 wt% of chlorhexidine or a salt thereof (expressed as chlorhexidine free base)
- at least 70 wt% of water,
- optionally 5 to 10 wt% of propylene glycol,
- optionally 1 to 10 wt% of glycerin, and
- optionally 1 to 5 wt% PEG40 hydrogenated castor oil,
wherein the wt% is expressed with respect to the total weight of the composition.

In a particular embodiment the oral care compositions are in the form of a tooth gel or toothpaste which comprises:
- 0.005 to 1 wt% of o-cymen-5-ol,
- 0.05 to 4 wt% of hydroxypropyl methylcellulose,
- 0.05 to 0.5 wt% of chlorhexidine or a salt thereof (expressed as chlorhexidine free base)
- 60 to 75 wt% of water,
- optionally 5 to 55 wt% of sorbitol,
- optionally 0.1 to 1.5 wt% xanthan gum,
- optionally 0.5 to 10 wt% of propylene glycol, and
- optionally 1 to 35 wt% of glycerin,
wherein the wt% is expressed with respect to the total weight of the composition.

The oral care compositions of the invention also comprise water. Preferably, water is present in an amount of at least 70 wt%, at least 75 wt%, or at least 80 wt%, wherein the wt% is expressed with respect to the total weight of the composition.

In a preferred embodiment, the oral care composition of the invention also comprise one or more ingredients selected from the group consisting of fluoride sources, surfactants, co-solvents, humectants, sweeteners, binders, mouth-feel agents, abrasives, desensitizing agents, other oral care active ingredients, pH modifying agents, chelating agents, other antimicrobial agents, flavoring agents, and colorants.

Fluoride ion sources include sodium fluoride, potassium fluoride, stannous fluoride, potassium monofluorophosphate, sodium monofluorophosphate, ammonium monofluorophosphate, sodium fluorosilicate, ammonium fluorosilicate, amine fluoride such as (N'-octadecyltrimethylendiamine-N,N,N'-tris(2-ethanol)-dihydrofluoride), ammonium fluoride, and combinations thereof. Preferably, the oral care composition of the invention may also contain a source of fluoride ions or fluorine-providing ingredient in amounts sufficient to supply 50 to 5000 ppm fluoride ion, e.g., from 100 to 1000 ppm fluoride ion. Fluoride ion sources may be added to the compositions of the invention at a level in the range of 0.001 wt% to 10 wt%, e.g., from 0.003 wt% to 5 wt%, or from 0.01 wt% to 1.2 wt%, wherein the wt% is expressed with respect to the total weight of the composition. However, it is to be understood that the weights of fluoride salts to provide the appropriate level of fluoride ion will obviously vary based on the weight of the counter ion in the salt, and one of skill in the art may readily determine such amounts. Preferred fluoride ion sources are sodium fluoride and sodium monofluorophsphate.

The oral care compositions of the present invention may optionally comprise a surfactant. The term "surfactant" refers to compounds that are capable of lowering the surface tension. Suitable surfactants include non-ionic, anionic, cationic and amphoteric surfactants. Any orally acceptable surfactant can be used.

Examples of non-ionic surfactants are block copolymers such as ethylene oxide and propylene oxide block copolymers (e.g. poloxamers), ethoxylated hydrogenated castor oils, polyoxyethylene sorbitan esters (e.g. polysorbates), fatty alcohol ethoxylates, alkylphenol ethoxylates, tertiary amine oxides, tertiary phosphine oxides, dialkylsulfoxides and the like. In a particular embodiment, the non-ionic surfactant is selected from ethylene oxide and propylene oxide block copolymers (e.g. poloxamers), ethoxylated hydrogenated castor oils, and mixtures thereof; preferably poloxamer 407, PEG 40 hydrogenated castor oil and mixtures thereof.

Examples of anionic surfactants are alkyl and alkenyl sulfates and their alkyl-ether derivatives, sarcosinates, isethionates and taurates, such as sodium lauryl sulfate, sodium coconut monoglyceride sulfonate, sodium lauryl sarcosinate, sodium lauryl isethionate, sodium laureth carboxylate, salts of C₈₋₂₀alkyl sulfates (such as sodium C₁₄-C₁₆ alkyl suflates), salts of C₈₋₂₀alkenyl sulfates (such as sodium C₁₄-C₁₆ alkenyl sulfates), and sodium dodecyl benzenesulfonate; preferably sodium lauryl sarcosinate and sodium salts of C₈₋₂₀alkyl sulfates (such as sodium C₁₄-C₁₆ alkyl suflates) and sodiumc salts of C₈-₂₀alkenyl sulfates (such as sodium C₁₄-C₁₆ alkenyl sulfates).

Examples of cationic surfactants are lauryl trimethylammonium chloride, cetyl pyridinium chloride, cetyl trimethylammonium bromide, diisobutylphenoxyethyl dimethylbenzylammonium chloride, coconut alkyltrimethylammonium nitrite, cetyl pyridinium fluoride and mixtures thereof.

Examples of amphoteric surfactants are myristyl betaine, palmityl betaine, lauryl betaine, cetyl betaine, stearyl betaine, lauramidopropyl betaine, cocoamidoethyl betaine, cocoamidopropyl betaine and the like; preferably cocamidopropylbetaine.

Preferred surfactants include polyoxyethylene hydrogenated castor oils, poloxamers (polypropylene glycol- polyethylene glycol block copolymers), salts of C₈₋₂₀alkyl sulfates, salts of C₈₋₂₀alkenyl sulfates, sodium lauryl sarcosinate, and cocoamidopropyl betaine.

One or more surfactants are optionally present in a total amount in the range of from 1 wt% to 70 wt%, wherein the wt% is expressed with respect to the total weight of the composition.

In addition to water, the oral care compositions of the invention may comprise one or more co-solvents such as glycerin, propylene glycol and/or ethanol. One or more co-solvents are optionally present in a total amount in the range of from 5 wt% to 20 wt%, wherein the wt% is expressed with respect to the total weight of the composition.

The oral care compositions of the present invention may optionally comprise a humectant, which is a substance that holds and retains moisture. Any orally acceptable humectant can be used, such as sugar alcohols, e.g. sorbitol and, and low molecular weight PEGs, such as PEG40. Most humectants also function as sweeteners. One or more humectants are optionally present in a total amount in the range of from 1 wt% to 70 wt%, wherein the wt% is expressed with respect to the total weight of the composition.

In addition to the humectants described above and to the glycyrrhizin derivatives described above, which also function as sweeteners (i.e. substances capable of providing a sweet taste), the oral care compositions of the present invention may optionally comprise a sweetener which is not a humectant. Examples of this type of sweeteners include artificial sweeteners such as xylitol, erythritol, sucralose, saccharin, acesulfame, neotam, neohesperidin, cyclamate, thaumatin, stevioside. These sweeteners (which are neither humectants not glycyrrhizin derivatives) may be present in amounts in the range of 0.001 wt% to 2 wt%, wherein the wt% is expressed with respect to the total weight of the composition.

The oral care compositions of the invention may contain a binder, which provides cohesion to the composition. Any conventional binder may be utilized, such as, carboxyvinyl polymers, carrageenans, starches, natural gums such as xanthan gum, karaya, gum arabic and tragacanth, and chitosan. One or more binders may be present in a total amount in the range 0.1 wt% to 1.5 wt%, wherein the wt% is expressed with respect to the total weight of the composition.

Mouth-feel agents that may be present in the oral care compositions of the invention and refer to materials which impart a desirable texture or other feeling during use of the composition. Such agents include bicarbonate salts, which may impart a clean feel to teeth and gums due to effervescence and release of carbon dioxide. Any orally acceptable bicarbonate can be used, such as alkali metal bicarbonates e.g. sodium and potassium bicarbonates, ammonium bicarbonate, and mixtures thereof. One or more bicarbonate salts are optionally present in a total amount in the range of 0.1 wt% to 5 wt%, wherein the wt% is expressed with respect to the total weight of the composition.

The oral care compositions of the present invention may also include an abrasive, which removes material from the teeth and gum surfaces and aids in mechanical tooth cleaning. The abrasive may be a silica abrasive such silica, sodium metaphosphate, potassium metaphosphate, tricalcium phosphate, dihydrated dicalcium phosphate, aluminum silicate, calcined alumina, bentonite or other siliceous materials, sodium chloride, or combinations thereof. One or more abrasives may be present in a total amount in the range from 10 wt% to 30 wt%, preferably from 15 wt% to 20 wt% %, wherein the wt% is expressed with respect to the total weight of the composition.

The oral care composition of the present invention may also comprise a desensitizing agents, which treat hypersensitivity by blocking dentin tubules when applied to a tooth. Examples of desensitizing agents are potassium salts such as potassium nitrate, potassium chloride, potassium bicarbonate, potassium citrate, and potassium oxalate. Such agents may be added in effective amounts, e.g., in a total amount typically in the range from 0.01 wt % to 5 wt%, wherein the wt% is expressed with respect to the total weight of the composition.

In addition to the oral care active ingredients mentioned above, other oral care active ingredients may be present in the compositions of the invention. Said oral care active ingredients may exert any beneficial effect to teeth and gums, such as reducing hypersensitivity of the teeth, reducing plaque accumulation, reducing or inhibiting demineralization and promote remineralization of the teeth, inhibiting microbial biofilm formation in the oral cavity, reducing or inhibiting gingivitis, promoting healing of sores, reducing levels of acid producing bacteria, increasing relative levels of non-cariogenic and/or non-plaque forming bacteria, reducing or inhibiting formation of dental caries, reducing, repairing or inhibiting pre-carious lesions of the enamel, treating, relieving or reducing dry mouth, cleaning the teeth and oral cavity, reducing erosion, whiten teeth, and reducing tartar build-up. Examples of said agents include alkali metal gluconate salts such as sodium gluconate, panthenol, vitamin E or a derivative thereof such as vitamin E acetate, α-bisabolol, malic acid, calcium glycerophosphate, etc. It is to be understood that the weights of these active ingredients may vary depending on the particular active ingredient, and one of skill in the art may readily determine such amounts. Typically each oral care active ingredient is optionally present in an amount in the range from 0.001 wt% to 1 wt%%, wherein the wt% is expressed with respect to the total weight of the composition.

The oral care compositions of the present invention may comprise a pH modifying agent, which is a compound that adjust and/or maintain pH. Such agents include acidifying agents to lower pH, basifying agents to raise pH, and buffering agents to control pH within a desired range. For example, one or more compounds selected from acidifying, basifying and buffering agents can be included to provide a pH of 4.5 to 10.5, preferably 5.5 to 8.5.

Any orally acceptable pH modifying agent can be used, including carboxylic, phosphoric and sulfonic acids and their salts (e.g., monopotassium phosphate, dipotassium phosphate, monosodium phosphate, trisodium phosphate, citric acid, monosodium citrate, disodium citrate, monosodium malate, etc.), alkali metal hydroxides such as sodium hydroxide, carbonates such as sodium carbonate, bicarbonates, sesquicarbonates, borates, silicates, imidazole and the like. One or more pH modifying agents are optionally present in a total amount effective to maintain the composition in an orally acceptable pH range.

The oral care compositions of the invention may optionally comprise a chelating agent, which forms complexes with metal ions, such as calcium, magnesium and iron. Examples of chelating agents are citric acid, succinic acid, gluconic acid, etidronic acid, galactaric acid, galacturonic acid, glucuronic acid, phytic acid, ethylenediaminetetraacetic acid (EDTA), methylglycine-N,N-diacetic acid (MGDA), N,N-Dicarboxymethyl glutamic acid (GLDA), and the respective salts of ammonium, sodium and potassium; poly-acrylic acids, polymers thereof and the respective salts of ammonium, sodium and potassium; and phosphoric acids and poly-phosphoric acids and the respective salts of ammonium, sodium and potassium. Preferably, the chelating agent is EDTA or an ammonium, sodium or potassium salt thereof. One or more chelating agents are optionally present in a total amount in the range of from 0.1 to 5 wt%, wherein the wt% is expressed with respect to the total weight of the composition.

Other antimicrobial agents may also be present in the oral care compositions of the invention. Antimicrobial agents inhibit the development of microorganism in the oral care composition. Examples of suitable antimicrobial agents are chlorhexidine, triclosan, quaternary ammonium compounds (such as benzalkonium chloride) or parabens (such as methyl or propyl paraben). One or more antimicrobial agents are optionally present in a total amount typically in the range from 0.001 wt% to 0.5 wt%, wherein the wt% is expressed with respect to the total weight of the composition.

The oral care compositions of the present invention may optionally comprise a flavoring agent that is added to modify the taste and the aroma of the oral care compositions. Flavoring agents that may be used in the compositions of the present invention include essential oils as well as various flavoring aldehydes, esters, alcohols, and similar materials. Examples of the essential oils include oils of spearmint, peppermint, aniseed, wintergreen, sassafras, clove, sage, eucalyptus, marjoram, cinnamon, lemon, lime, grapefruit, and orange. Also useful are compounds such as menthol, carvone, anethole and vainillin. Of these, the most commonly employed are the oils of peppermint and spearmint. One or more flavoring agents may be present in the oral care composition at a total concentration in the range of 0.01 wt% to 5 wt%, wherein the wt% is expressed with respect to the total weight of the composition.

The oral care composition of the invention may comprise at least one colorant, which is added to modify the colour of the compositions. Colorants herein include pigments, dyes, lakes and agents imparting a particular luster or reflectivity such as pearling agents. Any orally acceptable colorant can be used, such as titanium dioxide. One or more colorants are optionally present in a total amount in the range of from 0.0001 wt% to 5 wt%, wherein the wt% is expressed with respect to the total weight of the composition.

The oral care compositions of the invention may be in the form of mucoadhesive compositions such as a mouthwash, a toothpaste or a tooth gel.

In the context of the present invention, "mouthwash" is equivalent to "oral rinse", "mouth rinse" and "mouth bath" and designate liquid compositions which are suitable for oral hygiene, such as by reducing the microbial load in the oral cavity, in particular the bacterial plaque that causes cavities, gingivitis and bad breath. When using such compositions, they are typically maintained in the mouth passively and/or swished through the teeth by contraction of the perioral muscles and/or movement of the head, and may be gargled, and finally spit out.

In the context of the present invention, the term "toothpaste" refers to a semisolid preparation suitable for cleaning the teeth when used with a toothbrush. In the context of the present invention, the term "tooth gel" refers to a gel preparation suitable for cleaning the teeth when used with a toothbrush. Tooth gels are like toothpastes but clear or translucent in appearance. In particular, tooth gels do not comprise titanium dioxide whereas toothpastes comprise titanium dioxide.

In a particular embodiment, the oral care composition of the invention is a mouthwash and comprises water in an amount of at least 70 wt%, at least 75 wt%, or at least 80 wt%; preferably at least 75 wt% of water. The wt% is expressed with respect to the total weight of the composition.

In another particular embodiment, the oral care composition of the invention is a tooth gel or toothpaste and comprises water in an amount of at least 60 wt%of water. The wt% is expressed with respect to the total weight of the composition.

**In** another particular embodiment, the oral care composition of the invention is a tooth gel or toothpaste and comprises water in an amount of at least 60 wt% of water. The wt% is expressed with respect to the total weight of the composition.

The oral care compositions of the invention may be obtained by mixing the above mentioned ingredients.

### Uses of the compositions

As explained in the background section, o-cymen-5-ol has antimicrobial properties against different bacteria and has been reported to penetrate deep-inside plaque, which helps to control the development of dental plaque, calculus formation, bleeding gums, and malodour. o-cymen-5-ol also reduce the breath concentrations of volatile sulphur compounds (VSC), which are associated with halitosis, as well as having direct and indirect inhibitory effects on anaerobe mediated-VSC production.

Accordingly, a second aspect of the present invention is directed to the oral care compositions of the invention for use in maintaining the health of the oral cavity, in particular for use in the treatment and/or prevention of dental plaque formation, caries formation, calculus formation, halitosis, gingivitis, periodontitis, peri-implant mucositis and/or periimplantitis.

For the above purposes, the aqueous oral composition of the invention may be contacted with a surface of the oral cavity. Said surface of the oral cavity may be selected from the group consisting of one or more teeth, surfaces of the gums, surface of the tongue and combinations thereof. For performing said contacting, when the oral care composition of the invention a mouthwash, it is typically held in the oral cavity passively or swirled around the mouth by contraction of the perioral muscles and/or movement of the head, and may be gargled, and finally spit out. In the case of mouthwashes, the amount of composition dosed in the oral cavity to perform the activity is typically from 2 to 30 ml, preferably from 5 to 20 ml and most preferable from 10 to 15 ml. The contacting may be performed for any suitable amount of time, such as for less than thirty seconds, for thirty seconds or more, for about thirty seconds, for about forty seconds, for about one minute or more. For performing the contact with a surface of the oral cavity, when the oral care composition of the invention is a tooth gel or toothpaste, it is typically applied with a toothbrush and mechanical cleaning of the teeth and/or tongue is performed, preferably for at least two minutes, more preferably for at least three minutes. In the case of tooth gels and toothpastes, the amount of composition dosed to perform the activity is typically from 0.3 to 4.0 g, preferably from 0.5 to 2.5 g. The contacting may be carried out at least once daily, preferably at least twice daily, more preferably at least three times daily, such as 1, 2, 3 or 4 times per day. The oral cavity may belong to a human or animal, preferably a human.

The term "treatment", "treating" or "treat", as used herein, refers to means reversing, alleviating, inhibiting the progress of the disease or disorder these terms refer to, or one or more symptoms of such disease or condition.

The term "prevention", "preventing" or "prevent", as used herein, refers to avoiding the appearance of a certain disease or disorder. The prevention can be complete (e.g. the total absence of a disease or disorder). The prevention can also be partial, such that for example the occurrence of a disease or disorder in a subject is less than that which would have occurred without the administration of the oral care composition of the present invention. The prevention also includes reducing the risk of suffering the disease or disorder.

The term "dental plaque" refers to a fine film comprised of remains of food, bacteria and saliva. If dental plaque is not eliminated regularly, it sticks to the surface of the enamel, attacking tooth structures and producing a series of substances that cause the gum swelling, giving rise to gingivitis (inflammation of the gum), redness and probable gingival bleeding.

The term "calculus" or "tartar" refers to a form of hardened dental plaque. It is caused by precipitation of minerals from saliva and gingival crevicular fluid in plaque on the teeth. This process of precipitation kills the bacterial cells within dental plaque, but the rough and hardened surface that is formed provides an ideal surface for further plaque formation. This leads to calculus buildup, which compromises the health of the gingiva (gums). Calculus can form both along the gumline, where it is referred to as supragingival, and within the narrow sulcus that exists between the teeth and the gingiva, where it is referred to as subgingival.

The term "gingivitis" refers to an inflammation of the gums that gives rise to an increase in sensitivity and irritation and which in many cases is accompanied by bleeding. Its most frequent trigger is dental plaque or biofilm, consisting of a fine film comprised of remains of food, bacteria and saliva. If it is not treated in time, it can evolve to periodontitis (pyorrhoea), and even enter the tooth and damage its bone structure, leading to the irreversible loss of the tooth.

The term "periodontitis" refers to an inflammatory gingival process that spreads to the periodontal ligament which holds and fixes the tooth, and even the alveolar bone, destroying both tissues. Periodontal bags appear, which are an increase in the gap or space between the surface of the tooth and the internal part of the gum. This destructive process spreads until tooth stability is compromised. Due to the loss of fixation, in the end stages the teeth become separated and loose, losing their normal location, and finally the tooth irremediably falls out. *Streptococci, spirochetes* and *bacteroides* are found to be the possible pathogens responsible for the disease [Prasanth M., Dent Res J (Isfahan), 2011, 8(2), 85-94; Menendez A. et al., Oral Microbiology Immunology, 2005, 20(1), 31-34].

The term "halitosis" or "bad breath" refers to the presence of unpleasant odours that can be detected in exhaled air. Halitosis is a condition that affects many people. There are multiple types of halitosis and they are classified depending on the source or cause of this bad breath: physiological halitosis, pathological halitosis (oral cause, 87% of all halitosis, and extraoral cause), pseudohalitosis and halitophobia. Bad breath through an oral cause is therefore the most common. This type of halitosis is caused by the production and release of volatile compounds, mainly sulphur compounds. Other molecules such as the diamines (for example, cadaverine) are also related to halitosis. The volatile sulphur compounds are caused by bacteria that grow in the mouth in conditions of oxygen absence (anaerobes). These volatile sulphur compounds come from the metabolism of food bacteria or remain trapped in the mouth.

The term "caries" or "cavities" refers to the breakdown of teeth structure due to acids produced by bacteria. *Streptococcus mutans* is considered one of the main bacteria involved in this process. In addition, other microflora like *Escherichia coli* and *Candida* are also associated with active caries lesions [Prasanth M., Dent Res J (Isfahan), 2011, 8(2), 85-94]. The cavities may be a number of different colors from yellow to black. Symptoms may include pain and difficulty with eating. Complications may include inflammation of the tissue around the tooth, tooth loss, and infection or abscess formation. It is a disease characterized by a series of reactions that lead the teeth's hard tissues to soften, and which if it goes untreated, advances from the surface inwards, eventually leading to the irreversible destruction of the tooth. Caries normally goes unnoticed by the patient. A detailed exploration will detect stains or white chalk-like spots (areas where there is weakened enamel) or brown pigmentation on the tooth surface. This phase may be characterized by a certain sensitivity (strange sensation) to some foods, particularly sweets or hot or cold drinks, although usually there is no actual pain. When the caries advances and affects the pulp or nerve inside the tooth, pain will generally ensue, although if the affection is very slow it may destroy a large part of the nerve painlessly. When the affection is deep, abscesses may form (better known as gumboils, i.e. destroyed dental material and pus) and give rise to major inflammation and pain.

The term "peri-implant mucositis" refers to an inflammatory lesion of the soft tissues surrounding an endo-osseous implant in the absence of loss of supporting bone or continuing marginal bone loss. Peri-implant mucositis is caused by bacteria from dental biofilms, which disrupts the host-microbe homeostasis at the implant-mucosa interface, resulting in an inflammatory lesion. Biofilm removal is a prerequisite for the prevention and management of peri-implant mucositis. Peri-implant mucositis is considered a precursor for peri-implantitis.

The term "periimplantitis" refers to an inflammatory lesion of the mucosa that affects the surrounding tissue of the implant, predominately the supporting bone with loss of osseointegration. As peri-implant mucositis, it is also caused by the adhesion of pathogenic bacterial biofilms on the implant surface and peri-implant tissues.

In the context of the invention, the term "about" when characterizing a value refers to ±10% of said value, preferably ±5% of said value.

Preferably, the expression "one or more" refers to 1, 2, 3, 4, 5, 6, 7, 8, 9 or 10, more preferably to 1, 2, 3, 4 or 5, even more preferably to 1, 2 or 3.

The following examples are provided to illustrate the invention but should not be considered as limiting its scope that is defined by the appended claims.

### Examples

### Compositions

The mouthwash compositions of comparative examples 1 to 3 and of example 4 are prepared using the procedure detailed below:
In a reactor water is added followed by colorants, xylitol, sucralose and vainillin, the mixture was stirred for 15 minutes.

In a separate first mixing vessel the cellulosic polymer (such as hydroxypropyl methyl cellulose or hydroxyethyl cellulose) is dispersed in glycerine. This dispersion is added to the reactor and stirred.

In a second mixing vessel a mixture of propylenglycol, PEG-40 hydrogenated castor oil, menthol, neohesperidin DC, aroma and o-cymen-5ol is prepared under stirring until complete dissolution. The mixture of the second mixing vessel is then added to the content of the reactor mixing vessel and is stirred for 30 minutes.

A 20% aqueous solution of chlorhexidine digluconate is added to the reactor and is stirred for 30 minutes at high speed. The pH is then adjusted to 5.4-6.0 using sodium citrated and citric acid as needed and stirring for 20 minutes. The resulting mixture is filtered through a 5µm filter.

The mouth wash compositions of comparative examples 1-3, 5-7 and example 4 shown below do all comprise the following ingredients in common accounting for 18.3884 wt.% of the total compositions:
Xylitol 1 wt.%
Sucralose 0.13 wt.%
Vainillin 0.003 wt.%
Glycerin 8 wt.%
Neohesperidin DC 0.003 wt.%
Sodium citrate 0.05 wt.%
Citric acid monohydrate 0.0112 wt.%
Propylenglycol 8 wt.%
PEG-40 hydrogenated castor oil 1 wt.%
Flavour (1/074569) 0.11 wt.%
Menthol 0.08 wt.%
Colorant (80% E-124) 0.0012 wt.%

In addition, the compositions of comparative examples 1-3, 5-7 and example 4 contain the ingredients specified in table 1 below. The ensemble of the ingredients mentioned in the previous paragraph are is indicated in the table as "base composition".

**Table 1**

| *Ingredient* | *Comp. Ex. 1* | *Comp. Ex. 2* | *Comp. Ex.* 3 | *Ex. 4* | *Comp. Ex. 5* | *Comp. Ex. 6* | *Comp. Ex. 7* |
|---|---|---|---|---|---|---|---|
| Base composition | 18.3884 | | | | | | |
| Chlorhexidine digluconate (20%) | 0 | 0 | 0.636 | 0.636 | 0.636 | 0.636 | 0.636 |
| *o*-Cymen-5-ol | 0 | 0.1 | 0 | 0.1 | 0 | 0.1 | 0.1 |
| Hydroxypropyl methyl cellulose (BENECEL KM4 Pharma CR) | 0.10 | 0.10 | 0.10 | 0.10 | - | - | - |
| Hydroxy ethyl cellulose (NATROSOL M PHARMA) | - | - | - | - | 0.10 | 0.10 | - |
| Water | *q.s.* 100 | | | | | | |

The gel compositions of comparative examples 8 to 10 and of example 11 are prepared using the procedure detailed below:
Sorbitol and glycerine are added to a reactor. Then hydroxypropyl methyl cellulose is added gradually through the reactor mouth. The mixture is stirred with the paddles and the vacuum connected at a speed of 50 rpm for 10 minutes. After the 10 minutes of homogenization, the total of purified water, sucralose and vanillin are added. The paddle stirrer is the switched on at a speed of 50 rpm and the vacuum and turrax are switched on at 8000 rpm for 30 minutes. After this time, chlorhexidine digluconate and a premix prepared with propylene glycol, flavour, PEG 40 hydrogenated castor oil, menthol and o-cymen-5-ol are added. The mixture is the homogenised with paddle stirring at a speed of 50 rpm and the vacuum and turrax are switched on at a speed of 8000 rpm for 20 minutes.

The gel compositions of comparative examples 8 to 10 and example 11 shown below do all comprise the following ingredients in common accounting for 24.593 wt.% of the total compositions:
Propylenglycol 10 wt. %
Glycerin 7 wt. %
Sorbitol 7 wt.
PEG 40 hydrogenated castor oil 0.3 wt.%
Vainillin 0.003 wt. %
Menthol 0.1 wt. %
Flavour (1/074569) 0.12 wt. %
Sucralose 0.07 wt. %

In addition, the compositions of comparative examples 8 to 10 and example 11 contain the ingredients specified in table 2 below. The ensemble of the ingredients mentioned in the previous paragraph are is indicated in the table as "base composition".

**Table 2**

| *Ingredient* | *Comparative example 8* | *Comparative example 9* | *Comparative example 10* | *Example 11* |
|---|---|---|---|---|
| Base composition | 24.593 | 24.593 | 24.593 | 24.593 |
| Chlorhexidine digluconate (20%) | 0 | 1.060 | 0 | 1.060 |
| *o*-Cymen-5-ol | 0 | 0 | 0.1 | 0.1 |
| Hydroxypropyl methyl cellulose (BENECEL KM4 Pharma CR) | 3.2 | 3.2 | 3.2 | 3.2 |
| Water | *q.s.* 100 | *q.s.* 100 | *q.s.* 100 | *q.s.* 100 |

### Determination of the mucoadhesion capacity of formulations to the oral mucosa

The method described is based on the mucin adhesion model which is described, for example, in Madsen, F. et al. Journal of Controlled Release 50 (1998) 167-178 to assess the interaction between a given composition and mucin, which is a major component of the mucus of the oral mucose. This interaction is quantified by a relative viscosity measurement (*η*_{REL}).

### Materials

### 1. Preparation of a 10% mucin dispersion

5 grams of mucin type III are weighed in a beaker. Water is then added to a final weight of 50 grams. The beaker is stirred to complete dispersion until no lumps remain. The pH is adjusted with 0,5N NaOH or 1N HCl depending on whether pH is to be raised or lowered, to a final pH of 6.2 and 7.0.

### 2. Preparation of a 6% mucin dispersion

18 grams of the above mentioned 10% mucin dispersion are weighed in a beaker. Water is then added to a final weight of 30 grams. The beaker is stirred to complete dispersion until no lumps remain.

### 3. Preparation of a 3% mucin dispersion (M)

### Note: prepare the sample in duplicate

3 grams of the above mentioned 6 % mucin dispersion into a beaker. Water is then added to a final weight of 6 grams. The beaker is stirred for at least 30 minutes to complete homogenization.

### 4. Preparation of a dispersion of the composition to be tested (P)

### Note: prepare the sample in duplicate

3 grams of the composition to be tested are weighed in a beaker. Water is then added to a final weight of 6 grams. The beaker is stirred for at least 30 minutes to complete homogenization.

### 5. Preparation of mixture of mucin and the composition to be tested (P+M)

### Note: prepare the sample in duplicate

3 grams of the 3% mucin dispersion are weighed in a beaker. 3 grams of the composition to be tested are added. The beaker is stirred for at least 30 minutes to complete homogenization.

### Testing process

Using a Haake Mars viscosimeter viscosities at a shear rate of 0.2 s⁻¹ (for highly viscous products) or at a shear rate of 100 s⁻¹ (for non-highly viscous products) are calculated by building a line of viscosity vs. shear rate and interpolating the viscosity values (η_{P+M}, η_{P} and η_{M}) at a shear rate of 0.2 s⁻¹ (for highly viscous products) or 100 s⁻¹ (for non-highly viscous products) .

The line of viscosity vs. shear rate is made by measuring viscosity (two duplicates for each duplicate sample) employing the following conditions:

| | |
|---|---|
| Stabilisation 1: | 30 s at 0 s⁻¹ |
| Stabilisation 2: | 30 s at 0.1 s⁻¹ |
| Temperature: | 37°C |

Measurements of viscosity are made at the following shear rates which maintained during 30 s before taking the viscosity measure.
Shear rate 0.1 s⁻¹
Shear rate 1.0 s⁻¹
Shear rate 10.0 s⁻¹
Shear rate 100.0 s⁻¹
Shear rate 1000.0 s⁻¹

### Note:

To discriminate between highly viscous products and non-highly viscous products a first viscosity measurement at a shear rate of 100 s⁻¹ is carried out using a plate 35 sensor and a 0.3 mm groove.

When the viscosity of the formula to be tested measured at the above-mentioned conditions is higher than 150 mPa·s the product is considered a highly viscous product and the following conditions are used to build the viscosity/shear rate line and to interpolate the viscosity values at a shear rate of 0.2 s⁻¹:

| | |
|---|---|
| Sensor: | Plate 35 or equivalent |
| Groove: | 0.3 mm |

When the viscosity of the formula to be tested measured at the above-mentioned conditions is lower 150 mPa·s the product is considered a non-highly viscous product and following conditions are used to build the viscosity/shear rate line and to interpolate the value at a shear rate of 100 s⁻¹::

| | |
|---|---|
| Sensor: | 35/2° cone or equivalent |

### Calculations

From the viscosity readings (η_{P+M}, η_{P} and η_{M}) obtained from each sample (4 readings for each sample) of the mucin dispersion P, the compositions to be tested M and the mixture of mucin dispersion and composition to be tested (P+M) an average is taken and the following calculations are made to obtain the value *η*-relative and to be able to define the degree of mucoadhesivity. ${η}_{REL} = {η}_{P + M} / \left({η}_{P}+{η}_{M}\right)$ wherein η_{P+M} is the viscosity of the mixture of the product to be tested with the 3% mucin dispersion, η_{P} is the viscosity of the product to be tested and η_{M} is the viscosity of the 3% mucin dispersion.

The higher the value of *η*_{REL} ≥ the stronger is the interaction between mucin and the product under study. The expected in-vivo mucoadhesivity of the composition will be higher the higher the *η*_{REL} value.

### Results

The mucoadhesive behavior of the compositions of comparatives examples 1-3, 5-10 and that of the compositions of examples 4 and 11 determined using the above-describes methodology is reported in tables 3 and 4 below.

**Table 3**

| *Ingredient* | *Comp. Ex. 1* | *Comp. Ex. 2* | *Comp. Ex. 3* | *Ex. 4* | *Comp. Ex. 5* | *Comp. Ex. 6* | *Comp. Ex. 7* |
|---|---|---|---|---|---|---|---|
| *η*-relative | 1.15 | 1.05 | 0.96 | 1.19 | 1.10 | 1.12 | 1.03 |

**Table 4**

| *Ingredient* | *Comp. Ex. 8* | *Comp. Ex. 9* | *Comp. Ex. 10* | *Ex. 11* |
|---|---|---|---|---|
| *η*-relative | 1.15 | 1.05 | 0.96 | 1.19 |

The results summarized in the tables above show an unexpectedly good mucoadhesive behavior of the compositions of the invention (examples 4 and 11) when compared with similar compositions lacking at least one of the following ingredients: a) cellulosic polymer, b) o-cymen-5-ol, and (c) chlorhexidine or salts thereof (comparative examples 1-3, and 5-10).

## Claims

1. Oral care composition comprising:
- a cellulosic polymer that is hydroxypropyl methylcellulose,
- o-cymen-5-ol,
- from 0.05 wt% to 0.5 wt% of chlorhexidine or salts thereof expressed as chlorhexidine free base, and
- water in an amount of at least 60 wt%.

2. Oral care composition according to claim 1 wherein the cellulosic polymer is present at a concentration of from 0.05 to 4 wt% with respect to the total weight of the composition.

3. Oral care composition according to any one of the preceding claims wherein o-cymen-5-ol is present at a concentration of from 0.005 to 1 wt%, preferably from 0.01 to 0.5 wt%, more preferably from 0.02 to 0.2 wt%, most preferably from 0.05 to 0.1 wt% with respect to the total weight of the composition.

4. Oral care composition according to any one of the preceding claims not including any zinc salt having a solubility in water higher than 3.0·10⁴ mg/L at 25 °C.

5. Oral care composition according to any one of the preceding claims not including any glycyrrhizin derivative selected from glycyrrhizinic acid, glycyrrhetinic acid, salts and mixtures thereof.

6. Oral care composition according to any one of the preceding claims not including hydroxyacetophenone.

7. Oral care composition according to any one of the preceding claims wherein water is present in an amount of at least 70 wt%, at least 75 wt%, or at least 80 wt%, wherein the wt% is expressed with respect to the total weight of the composition.

8. Oral care composition according to any of the preceding claims further comprising one or more ingredients selected from the group consisting of fluoride ion sources, surfactants, co-solvents, humectants, sweeteners, binders, mouth-feel agents, abrasives, desensitizing agents, other oral care active ingredients, pH modifying agents, antimicrobial agents other than chlorhexidine or salts thereof, chelating agents, flavoring agents, and colorants.

9. Oral care composition according to any of the preceding claims in the form of a mouthwash, a tooth gel or a toothpaste.

10. Oral care composition according to any of the preceding claims in the form of a mouthwash which comprises:
- 0.005 to 1 wt% of o-cymen-5-ol,
- 0.05 to 4 wt% of the cellulosic polymer,
- 0.05 to 0.5 wt% of chlorhexidine or a salt thereof, expressed as chlorhexidine free base,
- at least 70 wt% of water,
- optionally 5 to 10 wt% of propylene glycol,
- optionally 1 to 10 wt% of glycerin, and
- optionally 1 to 5 wt% PEG40 hydrogenated castor oil,
wherein the wt% is expressed with respect to the total weight of the composition.

11. Oral care composition according to any of claims 1 to 9 in the form of a tooth gel of toothpaste which comprises:
- - 0.005 to 1 wt% of o-cymen-5-ol,
- 0.05 to 4 wt% of the cellulosic polymer,
- 0.05 to 0.5 wt% of chlorhexidine or a salt thereof, expressed as chlorhexidine free base,
- - 60 to 75 wt% of water,
- - optionally 5 to 55 wt% of sorbitol,
- - optionally 0.1 to 1.5 wt% xanthan gum,
- - optionally 0.5 to 10 wt% of propylene glycol, and
- - optionally 1 to 35 wt% of glycerin,
- wherein the wt% is expressed with respect to the total weight of the composition.

12. Oral care composition according to any of the preceding claims for use in the treatment and/or prevention of dental plaque formation, caries formation, calculus formation, halitosis, gingivitis, periodontitis, peri-implant mucositis and/or periimplantitis.

## Patentansprüche

1. Mundpflegezusammensetzung, umfassend:
- ein Cellulosepolymer, bei dem es sich um Hydroxypropylmethylcellulose handelt,
- *o*-Cymen-5-ol,
- 0,05 Gew.-% bis 0,5 Gew.-% Chlorhexidin oder Salze davon, ausgedrückt als freie Chlorhexidinbase, und
- Wasser in einer Menge von mindestens 60 Gew.-%.

2. Mundpflegezusammensetzung nach Anspruch 1, wobei das Cellulosepolymer in einer Konzentration von 0,05 bis 4 Gew.-%, bezogen auf das Gesamtgewicht der Zusammensetzung, vorliegt.

3. Mundpflegezusammensetzung nach einem der vorstehenden Ansprüche, wobei *o-*Cymen-5-ol in einer Konzentration von 0,005 bis 1 Gew.-%, vorzugsweise von 0,01 bis 0,5 Gew.-%, noch bevorzugter von 0,02 bis 0,2 Gew.-% und am meisten bevorzugt von 0,05 bis 0,1 Gew.-%, bezogen auf das Gesamtgewicht der Zusammensetzung, vorliegt.

4. Mundpflegezusammensetzung nach einem der vorstehenden Ansprüche, die kein Zinksalz enthält, dessen Löslichkeit in Wasser bei 25 °C höher als 3,0·10⁴ mg/l ist.

5. Mundpflegezusammensetzung nach einem der vorstehenden Ansprüche, die kein Glycyrrhizin-Derivat enthält, das aus Glycyrrhizinsäure, Glycyrrhetinsäure, Salzen und Mischungen davon ausgewählt ist.

6. Mundpflegezusammensetzung nach einem der vorstehenden Ansprüche, die kein Hydroxyacetophenon enthält.

7. Mundpflegezusammensetzung nach einem der vorstehenden Ansprüche, wobei Wasser in einer Menge von mindestens 70 Gew.-%, mindestens 75 Gew.-% oder mindestens 80 Gew.-% vorhanden ist, wobei die Gew.-% bezogen auf das Gesamtgewicht der Zusammensetzung angegeben sind.

8. Mundpflegezusammensetzung nach einem der vorstehenden Ansprüche, die ferner einen oder mehrere Inhaltsstoffe umfasst, ausgewählt aus der Gruppe bestehend aus Fluoridionenquellen, Tensiden, Co-Lösungsmittel, Feuchthaltemitteln, Süßungsmitteln, Bindemitteln, Mitteln zur Verbesserung des Mundgefühls, Schleifmitteln, desensibilisierenden Mitteln, anderen Wirkstoffen für die Mundpflege, pH-Modifikatoren, antimikrobiellen Mitteln, die kein Chlorhexidin oder Salze davon sind, Chelatbildnern, Aromastoffen und Farbstoffen.

9. Mundpflegezusammensetzung nach einem der vorstehenden Ansprüche in Form eines Mundwassers, eines Zahngels oder einer Zahnpasta.

10. Mundpflegezusammensetzung nach einem der vorstehenden Ansprüche in Form eines Mundwassers, die umfasst:
- 0,005 bis 1 Gew.-% *o*-Cymen-5-ol,
- 0,05 bis 4 Gew.-% des Cellulosepolymers,
- 0,05 bis 0,5 Gew.-% Chlorhexidin oder eines Salzes davon, ausgedrückt als freie Chlorhexidinbase,
- mindestens 70 Gew.-% Wasser,
- gegebenenfalls 5 bis 10 Gew.-% Propylenglykol,
- gegebenenfalls 1 bis 10 Gew.-% Glycerin und
- gegebenenfalls 1 bis 5 Gew.-% PEG40-hydriertes Rizinusöl,
wobei die Gew.-% bezogen auf das Gesamtgewicht der Zusammensetzung angegeben sind.

11. Mundpflegezusammensetzung nach einem der Ansprüche 1 bis 9 in Form eines Zahngels oder einer Zahnpasta, die umfasst:
- 0,005 bis 1 Gew.-% *o*-Cymen-5-ol,
- 0,05 bis 4 Gew.-% des Cellulosepolymers,
- 0,05 bis 0,5 Gew.-% Chlorhexidin oder eines Salzes davon, ausgedrückt als freie Chlorhexidinbase,
- - 60 bis 75 Gew.-% Wasser,
- - gegebenenfalls 5 bis 55 Gew.-% Sorbit,
- - gegebenenfalls 0,1 bis 1,5 Gew.-% Xanthan,
- - gegebenenfalls 0,5 bis 10 Gew.-% Propylenglykol und
- - gegebenenfalls 1 bis 35 Gew.-% Glycerin,
- wobei die Gew.-% bezogen auf das Gesamtgewicht der Zusammensetzung angegeben sind.

12. Mundpflegezusammensetzung nach einem der vorstehenden Ansprüche zur Verwendung bei der Behandlung und/oder Vorbeugung von Zahnbelagbildung, Kariesbildung, Zahnsteinbildung, Mundgeruch, Gingivitis, Parodontitis, periimplantärer Mukositis und/oder Periimplantitis.

## Revendications

1. Composition de soins bucco-dentaires comprenant :
- un polymère cellulosique qui est l'hydroxypropylméthylcellulose,
- l'*o*-cymen-5-ol,
- de 0,05 % en poids à 0,5 % en poids de chlorhexidine ou de sels de celle-ci exprimés en base libre de chlorhexidine, et
- de l'eau en une quantité d'au moins 60 % en poids.

2. Composition de soins bucco-dentaires selon la revendication 1, dans laquelle le polymère cellulosique est présent à une concentration de 0,05 à 4 % en poids par rapport au poids total de la composition.

3. Composition de soins bucco-dentaires selon l'une quelconque des revendications précédentes, dans laquelle l'*o*-cymen-5-ol est présent à une concentration de 0,005 à 1 % en poids, de préférence de 0,01 à 0,5 % en poids, plus préférentiellement de 0,02 à 0,2 % en poids, très préférentiellement de 0,05 à 0,1 % en poids par rapport au poids total de la composition.

4. Composition de soins bucco-dentaires selon l'une quelconque des revendications précédentes ne comprenant aucun sel de zinc ayant une solubilité dans l'eau supérieure à 3,0·10⁴ mg/L à 25 °C.

5. Composition de soins bucco-dentaires selon l'une quelconque des revendications précédentes ne comprenant pas de dérivé de glycyrrhizine choisi parmi l'acide glycyrrhizinique, l'acide glycyrrhétinique, leurs sels et leurs mélanges.

6. Composition de soins bucco-dentaires selon l'une quelconque des revendications précédentes ne comprenant pas d'hydroxyacétophénone.

7. Composition de soins bucco-dentaires selon l'une quelconque des revendications précédentes dans laquelle l'eau est présente en une quantité d'au moins 70 % en poids, d'au moins 75 % en poids, ou d'au moins 80 % en poids, dans laquelle le % en poids est exprimé par rapport au poids total de la composition.

8. Composition de soins bucco-dentaires selon l'une quelconque des revendications précédentes comprenant en outre un ou plusieurs ingrédients choisis dans le groupe constitué par les sources d'ions fluorure, les tensioactifs, les co-solvants, les humectants, les édulcorants, les liants, les agents de sensation en bouche, les abrasifs, les agents désensibilisants, d'autres principes actifs de soins bucco-dentaires, les agents modificateurs de pH, les agents antimicrobiens autres que la chlorhexidine ou ses sels, les agents chélatants, les agents aromatisants et les colorants.

9. Composition de soins bucco-dentaires selon l'une quelconque des revendications précédentes se présentant sous forme de bain de bouche, de gel dentaire ou de dentifrice.

10. Composition de soins bucco-dentaires selon l'une quelconque des revendications précédentes sous la forme d'un bain de bouche qui comprend :
- 0,005 à 1 % en poids d'*o*-cymen-5-ol,
- 0,05 à 4 % en poids du polymère cellulosique,
- 0,05 à 0,5 % en poids de chlorhexidine ou d'un sel de celle-ci, exprimé en base libre de chlorhexidine,
- au moins 70 % en poids d'eau,
- optionnellement 5 à 10 % en poids de propylène glycol,
- optionnellement 1 à 10 % en poids de glycérine, et
- optionnellement 1 à 5 % en poids d'huile de ricin hydrogénée PEG40,
dans laquelle le % en poids est exprimé par rapport au poids total de la composition.

11. Composition de soins bucco-dentaires selon l'une quelconque des revendications 1 à 9 se présentant sous forme de gel dentaire ou de dentifrice qui comprend :
- - 0,005 à 1 % en poids d'*o*-cymen-5-ol,
- 0,05 à 4 % en poids du polymère cellulosique,
- 0,05 à 0,5 % en poids de chlorhexidine ou d'un sel de celle-ci, exprimé en base libre de chlorhexidine,
- 60 à 75 % en poids d'eau,
- - optionnellement 5 à 55 % en poids de sorbitol,
- - optionnellement 0,1 à 1,5 % en poids de gomme xanthane,
- - optionnellement 0,5 à 10 % en poids de propylène glycol, et
- - optionnellement 1 à 35 % en poids de glycérine,
- dans laquelle le % en poids est exprimé par rapport au poids total de la composition.

12. Composition de soins bucco-dentaires selon l'une quelconque des revendications précédentes pour utilisation dans le traitement et/ou la prévention de la formation de plaque dentaire, de la formation de caries, de la formation de tartre, de l'halitose, de la gingivite, de la parodontite, de la mucosite péri-implantaire et/ou de la péri-implantite.
